(19) Europäisches Patentamt
European Patent Office
Office européen des brevets

(11) Veröffentlichungsnummer: **0 480 279 A1**

(12) **EUROPÄISCHE PATENTANMELDUNG**

(21) Anmeldenummer: **91116653.6**

(51) Int. Cl.⁵: **C07D 213/51**

(22) Anmeldetag: **30.09.91**

(30) Priorität: **08.10.90 DE 4031803**

(43) Veröffentlichungstag der Anmeldung:
**15.04.92 Patentblatt 92/16**

(84) Benannte Vertragsstaaten:
**BE CH DE FR GB LI NL**

(71) Anmelder: **BASF Aktiengesellschaft**
**Carl-Bosch-Strasse 38**
**W-6700 Ludwigshafen(DE)**

(72) Erfinder: **Hermeling, Dieter, Dr.**
**Viernheimer Strasse 8**
**W-6710 Frankenthal(DE)**
Erfinder: **Steckhan, Eberhard, Prof. Dr.**
**Jungholzweg 26**
**5309 Meckenheim(DE)**
Erfinder: **Magdi, Abdel Azzem, Dr.**
**3, Abdel Salam Badr E1 Dim St., El Zahra**
**Misr E1 Kadema, 11441 Cairo(EG)**

(54) **Verfahren zur Herstellung von Pyridinacylalen.**

(57) Verfahren zur Herstellung von Pyridinacylalen der allgemeinen Formel I

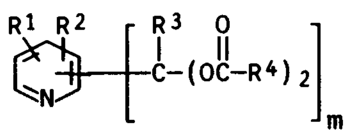

(I),

in der

- $R^1$ und $R^2$ unabhängig voneinander Wasserstoff, $C_1$- bis $C_{20}$-Alkyl, $C_1$- bis $C_{20}$-Alkoxy, Aryl, Aryloxy, Halogen, Amido, Carboxy, Cyano, $C_2$- bis $C_{20}$-Alkoxycarbonyl, $C_2$- bis $C_{12}$-Alkylcarbonyl oder gemeinsam eine gegebenenfalls durch $C_1$- bis $C_8$-Alkyl, $C_1$- bis $C_8$-Alkoxy, Aryl, Aryloxy, Halogen, Amido, Carboxy, Cyano oder $C_2$- bis $C_{20}$-Alkoxycarbonyl substituierte $(CH_2)_p$- oder $(CH=CH)_q$-Gruppe sein kann, wobei p für 1 bis 10 und q für 2 bis 4 steht,
- $R^3$ Wasserstoff, ein $C_1$- bis $C_{20}$-Alkyl, $C_3$- bis $C_{20}$-Cycloalkyl oder Aryl,
- $R^4$ ein $C_1$- bis $C_{12}$-Alkyl bedeutet,
- m für 1 oder 2 steht,

indem man Pyridinderivate der allgemeinen Formel II

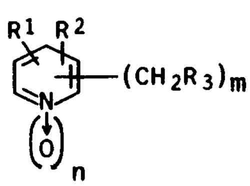

(II),

in der $R^1$, $R^2$, $R^3$ und m die obengenannten Bedeutungen haben und n für 0 oder 1 steht, mit einem Carbonsäureanhydrid der Formel $(R^4CO)_2O$, wobei $R^4$ die obengenannten Bedeutungen hat, in Gegenwart eines Übergangsmetallsalzes elektrochemisch umsetzt.

EP 0 480 279 A1

Die vorliegende Erfindung berifft ein neues Verfahren zur Herstellung von Pyridinacylalen sowie deren Verwendung zur Herstellung von Pyridincarbonylverbindungen.

Alkylaromaten lassen sich mit diversen Oxidationsmitteln wie Chromsäure, Salpetersäure und Permanganat in der Seitenkette oxidieren. In der Regel werden dabei die Alkylketten abgebaut, so daß die Alkylaromaten wie z.B. Methylaromaten zu aromatischen Carbonsäuren oxidiert werden. Dies trifft auch auf alkylsubstituierte Heteroaromaten zu. So werden z.B. Alkyl- oder Alkyliden-Pyridine nach DD-A-248 119 mit $KMnO_4$ zu Pyridincarbonsäuren oxidiert. Auch mit Kobalt- oder Mangansalzen katalysierte Sauerstoffoxidationen von Methylpyridinen führen nach Pakistan J. Sci. Ind. Res. 29, 336-337 (1986) oder DE-A-22 42 386 zu Pyridincarbonsäuren. Nach Pakistan J.Sci Ind. Res. 30, 182-184 (1987) läßt sich diese Oxidation auch direkt mit Kobalt-III-acetat in Essigsäure durchführen; die Ausbeute kann durch zusätzliche Anwesenheit von Sauerstoff gesteigert werden. Das Kobalt-III-acetat wird in diesem Fall in einem ex-Zell-Prozeß durch anodische Oxidation des leicht zugänglichen Kobalt-II-acetats hergestellt. Auch die direkte anodische Oxidation von Methylpyridinen im wäßrig sauren Milieu führt z.B. nach Trans. Soc. Advan. Electrochem. Sci. Technol., 97-99 (1971) oder Bull. Chem. Soc. Jpn. 7, 69-72 (1932) oder US-A-4,482,439 zu Pyridincarbonsäuren. Eine Oxidation von Methylpyridinen zu Pyridincarbaldehyden ist schwierig, da die Aldehyde sehr leicht zu Carbonsäuren weiteroxidiert werden. Dennoch gibt es einige wenige Methoden zur Realisierung dieses Syntheseschrittes. Am gebräuchlichsten ist eine Selendioxid-Oxidation, die z.B. aus Heterocycles 7, 347-352 (1977) oder Z. Naturforsch. B 34, 306-312 (1979) bekannt ist. Da $SeO_2$ nicht nur giftig, sondern wie in der Regel alle selektiven Oxidationsmittel auch sehr teuer ist, kommt eine technische Anwendung nicht in Frage.

Kostengünstigere technische Verfahren zur Herstellung aromatischer Aldehyde aus Toluolderivaten bedienen sich häufig der Schutzgruppenchemie, d.h. die durch Oxidation gebildeten Aldehyde werden in situ wie z.B. in Org. Synth. Coll. Vol. IV, 713-715 (1963) beschrieben, z.B. in Form ihrer Diacetate abgefangen und nach beendeter Reaktion zu den Aldehyden hydrolysiert. Methylpyridine werden wie z.B. in J.Am. Chem. Soc. 76, 1286-1290 (1954), Liebigs Ann. Chem. 1982, 1615-1622 oder US-A-4,634,711 beschrieben, zu N-Oxiden oxidiert. Die N-Oxide wiederum lassen sich in einem zweiten Reaktionschritt in Gegenwart von Acetanhydrid zu (Acetoxymethyl)-pyridinen umlagern. Eine zweite Oxidation dieser Produkte führt erneut zu N-Oxiden, die zu (Diacetoxymethy)-pyridinen umgelagert und anschließend zu Pyridincarbaldehyden verseift werden können, wie z.B. aus Liebigs Ann. Chem., 1615-1622 (1982) oder Khim. Geterocikl. Soed., 738-743 (1965) bekannt ist. Eine direkte Oxidation von Pyridinen zu den entsprechenden seitenkettenoxidierten $\alpha,\alpha$-Diacetaten ist nicht bekannt. Bekannt ist nach Heterocycles 5, 331-337 (1976) lediglich eine Seitenkettenoxidation von 2-Methylchinolin-N-oxid zu $\alpha,\alpha$-Diacetoxymethylchinolin mit hochgiftigem Thalliumtriacetat oder Bleitetraacetat.

Der vorliegenden Erfindung lag daher die Aufgabe zugrunde, den zuvor genannten Nachteilen abzuhelfen.

Demgemäß wurde ein neues und verbessertes Verfahren zur Herstellung von Pyridinacylalen der allgemeinen Formel I

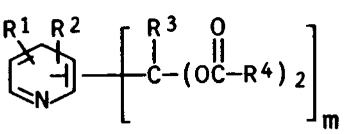

(I),

in der

| | |
|---|---|
| $R^1$ und $R^2$ | unabhängig voneinander Wasserstoff, $C_1$- bis $C_{20}$-Alkyl, $C_1$- bis $C_{20}$-Alkoxy, Aryl, Aryloxy, Halogen, Amido, Carboxy, Cyano, $C_2$- bis $C_{20}$-Alkoxycarbonyl, $C_2$- bis $C_{12}$-Alkylcarbonyl, oder gemeinsam eine gegebenenfalls durch $C_1$- bis $C_8$-Alkyl, $C_1$- bis $C_8$-Alkoxy, Aryl, Aryloxy, Halogen, Amido, Carboxy, Cyano oder $C_2$- bis $C_{20}$-Alkoxycarbonyl substituierte $(CH_2)_p$- oder $(CH=CH)_q$-Gruppe sein kann, wobei p für 1 bis 10 und q für 2 bis 4 steht, |
| $R^3$ | Wasserstoff, ein $C_1$- bis $C_{20}$-Alkyl, $C_3$- bis $C_{20}$-Cycloalkyl oder Aryl, |
| $R^4$ | ein $C_1$- bis $C_{12}$-Alkyl bedeutet, |
| m | für 1 oder 2 steht, |

gefunden, welches dadurch gekennzeichnet ist, daß man Pyridinderivate der allgemeinen Formel II

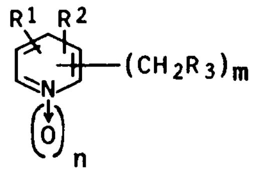

(II),

in der $R^1$, $R^2$, $R^3$ und m die obengenannten Bedeutungen haben und n für 0 oder 1 steht, mit einem Carbonsäureanhydrid der Formel $(R^4CO)_2O$, wobei $R^4$ die obengenannte Bedeutung hat, in Gegenwart eines Übergangsmetallsalzes elektrochemisch umsetzt.

Die elektrochemischen Oxidationen können sowohl in geteilter als auch in ungeteilter Zelle durchgeführt werden, bevorzugt wird eine ungeteilte Zelle verwendet. Die Elektrolytzusammensetzung kann in weiten Grenzen variiert werden.

Im speziellen hat der Elektrolyt beispielsweise folgende Zusammensetzung:
1 bis 40 Gew.%, vorzugsweise 2 bis 30 Gew.% Pyridinderivate der allgemeinen Formel II,
0,1 bis 20 Gew.%, vorzugsweise 0,1 bis 10 Gew.% Übergangsmetallsalze,
0 bis 30 Gew.%, vorzugsweise 0 bis 20 Gew.% eines Hilfselektrolyten,
0 bis 40 Gew.%, vorzugsweise 0 bis 30 Gew.% einer Carbonsäure $R^4$-COOH,
10 bis 98,9 Gew.%, vorzugsweise 20 bis 95 Gew.% eines Carbonsäureanhydrids $(R^4CO)_2O$.

Vorzugsweise wird die Elektrooxidation bei Stromdichten von 0,1 bis 200 mA/cm$^2$ durchgeführt. Als Anodenmaterialien eignen sich beispielsweise Edelmetalle wie Platin oder Oxide wie Ruthenium- oder Chromoxid oder $RuO_x/TiO_x$-Mischoxide und bevorzugt Graphit. Als Kathodenmaterialien kommen in der Regel Eisen, Stahl, Nickel und Edelmetalle wie Platin und bevorzugt Graphit in Betracht.

Die Aufarbeitung erfolgt in an sich bekannter Weise; vorzugsweise erfolgt die Reinisolierung durch Extraktion, Destillation, durch chromatographische Reinigung oder durch Kristallisation.

Die Oxidationen werden in Gegenwart von Übergangsmetallsalzen wie Cer-, Cobalt-, Chrom-, Kupfer-, Eisen-, Mangan-, Molybdän-, Titan-, Vanadium- oder Wolframsalzen, bevorzugt Cobalt-, Mangan-, Molybdän-, Vanadium- oder Wolframsalzen und eines Carbonsäureanhydrids wie z.B. Acetanhydrid, Propionsäureanhydrid, Butansäureanhydrid, Valeriansäureanhydrid, Hexansäureanhydrid, Heptansäureanhydrid oder Octansäureanhydrid, bevorzugt Essigsäureanhydrid oder Propionsäureanhydrid, durchgeführt.

Als Salze eignen sich beispielsweise Halogenide, wie Fluoride, Chloride, Bromide und Iodide, Sulfate, Nitrate, $C_2$-$C_{13}$-Carboxylate wie Acetate, Propionate und Butyrate, Phosphate, Fluoroborate, oder Hydroxide. Ferner eignen sich Tetrafluoroborate, Tetraalkylammoniumsalze wie Tetraethylammoniumbenzosulfonat, Perchlorate wie $LiClO_4$, Methylate wie Natriummethylat

Die genannten Übergangsmetallsalze können in ihrer niedrigsten Oxidationsstufe und somit in ihrer preisgünstigsten Form eingesetzt werden. Der Kostenaufwand wird noch weiter reduziert, indem diese Salze nur in katalytischen Mengen eingesetzt werden müssen.

Um eine für die Elektrolyse ausreichende Leitfähigkeit zu gewährleisten, wird gegebenenfalls ein zusätzlicher Hilfselektrolyt zugesetzt. Dabei kommen die in der Elektrochemie gebräuchlichen Hilfselektrolyte wie z.B. Tetrafluoroborate, Tetraalkylammoniumsalze wie Tetraethylammoniumbenzosulfonat, Perchlorate wie $LiClO_4$, Methylate wie Natriummethylat, Carbonsäuresalze wie Kaliumacetat, Natriumacetat, Hydroxide wie Kaliumhydroxid, Natriumhydroxid in Frage. Die Oxidationen werden bei Temperaturen zwischen 40 und 150 °C, vorzugsweise zwischen 60 und 120 °C sowohl drucklos (Normaldruck, Atmosphärendruck) als auch unter einem Druck von 0,1 bis 50 bar, bevorzugt 1,5 bis 10 bar, durchgeführt.

Als Substituenten $R^1$ bis $R^4$ und Indices n, m, p, q in den Formeln I, II und III kommen solche Reste in Betracht, die unter den Reaktionsbedingungen weitestgehend inert sind und unabhängig voneinander folgende Bedeutung haben können:

$R^1$, $R^2$ - Wasserstoff,

- verzweigtes oder unverzweigtes $C_1$- bis $C_{20}$-Alkyl, vorzugsweise verzweigtes oder unverzweigtes $C_1$- bis $C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, tert.-Pentyl, n-Hexyl, n-Heptyl, n-Octyl, n-Nonyl, n-Decyl, n-Undecyl und n-Dodecyl,
- $C_1$- bis $C_{20}$-Alkoxy, vorzugsweise $C_1$- bis $C_{12}$-Alkoxy, wie Methoxy, Ethoxy, Propoxy, Butoxy, tert.-Butyloxy, Pentyloxy, Hexyloxy, Heptyloxy, Octyloxy, Nonyloxy, Decyloxy, Undecyloxy und Dodecyloxy,
- Aryl wie Phenyl, 1-Naphthyl und 2-Naphthyl, bevorzugt Phenyl,
- Aryloxy wie Phenoxy, 1-Naphthyloxy und 2-Naphthyloxy, bevorzugt Phenoxy,
- Halogen wie Fluor, Chlor, Brom und Jod, bevorzugt Fluor, Chlor und Brom,

- Amido,
- Carboxy,
- Cyano,
- $C_2$- bis $C_{20}$-Alkoxycarbonyl, bevorzugt $C_2$- bis $C_8$-Alkoxycarbonyl, wie Methoxycarbonyl, Ethoxycarbonyl, Propoxycarbonyl, Butoxycarbonyl und tert.-Butoxycarbonyl,
- $C_2$- bis $C_{12}$-Alkylcarbonyl, vorzugsweise $C_2$- bis $C_8$-Alkylcarbonyl wie Acetyl, Propionyl, Butyryl, Valeroyl, Hexanoyl, Heptanoyl und Octanoyl.

$R^1$ und $R^2$ gemeinsam eine gegebenenfalls durch $C_1$- bis $C_8$-Alkyl, bevorzugt $C_1$- bis $C_4$-Alkyl wie Methyl, Ethyl, n-Propyl, Isopropyl, n-Butyl, iso-Butyl, sec.-Butyl und tert.-Butyl, $C_1$- bis $C_8$-Alkoxy, bevorzugt $C_1$- bis $C_4$-Alkoxy wie Methoxy, Ethoxy, n-Propoxy, iso-Propoxy, n-Butoxy, iso-Butoxy, sec.-Butoxy und tert.-Butyloxy, Aryl wie Phenyl, Aryloxy wie Phenoxy, Halogen wie Fluor, Chlor und Brom, Amido, Carboxy, Cyano oder $C_2$- bis $C_{20}$-Alkoxycarbonyl, wie Methoxycarbonyl, Ethoxycarbonyl, Propoxycarbonyl, Butoxycarbonyl, substituierte $(CH_2)_p$- oder $(CH=CH)_q$-Gruppe sein kann,

p 1 bis 10, vorzugsweise 3 bis 8,

q 2 bis 4,

$R^3$ - Wasserstoff,

- $C_1$- bis $C_{20}$-Alkyl, vorzugsweise $C_1$- bis $C_{12}$-Alkyl wie Methyl, Ethyl, n-Propyl, iso-Propyl, n-Butyl, sek.-Butyl, tert.-Butyl, Pentyl, Hexyl, Heptyl, Octyl, Nonyl, Decyl, Undecyl und Dodecyl,
- $C_3$- bis $C_{20}$-Cycloalkyl, vorzugsweise $C_3$- bis $C_{12}$-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl,
- Aryl, wie Phenyl, 1-Naphthyl und 2-Naphthyl, vorzugsweise Phenyl,

$R^4$ - $C_1$- bis $C_{12}$-Alkyl, vorzugsweis $C_1$- bis $C_8$-Alkyl wie Methyl, Ethyl, Propyl, Butyl, Pentyl, Hexyl, Heptyl und Octyl,

n 0 oder 1,

m 1 oder 2.

Die Pyridinacylale der allgemeinen Formel I stellen Vorstufen für die entsprechenden Pyridincarbonylverbindungen der allgemeinen Formel III dar. Die Verbindungen der Formel I zeichnen sich dadurch aus, daß sie oxidationsstabil sind und sich deshalb als stabile Lagersubstanzen der Verbindungen der Formel III eignen. Die Verbindungen der Formel III, die sehr leicht und nahezu quantitativ durch Hydrolyse der Verbindungen der Formel I zugänglich sind, stellen z.B. nach US-A-4,696,938, JP-A 57/146 756 oder EP-A-104 876 wichtige Vorstufen für Pflanzenschutzmittel oder z.B. nach J. Med. Chem. 16, 1096 bis 1101 (1973), US-A-4,634,711, EP-A-183 002 oder J. Org. Chem. 48, 4852 bis 4860 (1983) wichtige Pharmavorstufen dar.

Beispiele

Beispiel 1

Elektrooxidation von 4-Methylpyridin

Apparatur: ungeteilte Becherglaszelle (200ml) mit Kühlmantel
Anode: Graphit
Elektrolyt: 0,93 g (10 mmol) 4-Methylpyridin, 0,177 g (1 mmol) Co-II-acetat, 0,98 g (10 mmol) Kaliumacetat und 150 g Acetanhydrid
Kathode: Graphit
Stromdichte: 2mA/cm$^2$
Elektrolysetemperatur: 80 °C
Elektrolyse mit 4 F/mol 4-Methylpyridin.

Nach Beendigung der Elektrolyse wird das Lösungsmittel abdestilliert, das Rohprodukt in Chloroform aufgenommen und filtriert. Die Chloroformphase wurde säulenchromatographisch mit Chloroform/Methanol (100:1) als Laufmittel gereinigt. Man erhält 1,15 g (55 %) 4-(Diacetoxymethyl)-pyridin.

Beispiel 2

Elektrooxidation von 4-Methylpyridin-N-oxid

4-Methylpyridin-N-oxid wird unter den in Beispiel 1 beschriebenen Bedingungen oxidiert und aufgearbeitet. Elektrolyt: 1,09 g (10 mmol) 4-Methylpyridin-N-oxid, 0,177 g (1 mmol) Co-II-acetat und 150 g Acetanhydrid. Nach Aufarbeitung und Reinigung werden 1,42 g (68 %) 4-(Diacetoxymethyl)-pyridin erhalten.

Beispiel 3

Elektrooxidation von 2-Methylpyridin

2-Methylpyridin wird unter den in Beispiel 1 beschriebenen Bedingungen oxidiert und aufgearbeitet. Elektrolyt: 0,93 g (10 mmol) 2-Methylpyridin, 0,177 g (1 mmol) Co-II-acetat, 0,98 g (10 mmol) Kaliumacetat und 150 g Acetanhydrid.
Nach Aufarbeitung und Reinigung werden 0,21 g (10 %) 2-(Diacetoxymethyl)-pyridin erhalten.

**Patentansprüche**

1. Verfahren zur Herstellung von Pyridinacylalen der allgemeinen Formel I

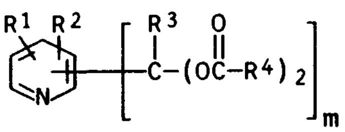
(I),

in der

| | |
|---|---|
| $R^1$ und $R^2$ | unabhängig voneinander Wasserstoff, $C_1$- bis $C_{20}$-Alkyl, $C_1$- bis $C_{20}$-Alkoxy, Aryl, Aryloxy, Halogen, Amido, Carboxy, Cyano, $C_2$- bis $C_{20}$-Alkoxycarbonyl, $C_2$- bis $C_{12}$-Alkylcarbonyl oder gemeinsam eine gegebenenfalls durch $C_1$- bis $C_8$-Alkyl, $C_1$- bis $C_8$-Alkoxy, Aryl, Aryloxy, Halogen, Amido, Carboxy, Cyano oder $C_2$- bis $C_{20}$-Alkoxycarbonyl substituierte $(CH_2)_p$- oder $(CH=CH)_q$-Gruppe sein kann, wobei p für 1 bis 10 und q für 2 bis 4 steht, |
| $R^3$ | Wasserstoff, ein $C_1$- bis $C_{20}$-Alkyl, $C_3$- bis $C_{20}$-Cycloalkyl oder Aryl, |
| $R^4$ | ein $C_1$- bis $C_{12}$-Alkyl bedeutet, |
| m | für 1 oder 2 steht, |

dadurch gekennzeichnet, daß man Pyridinderivate der allgemeinen Formel II

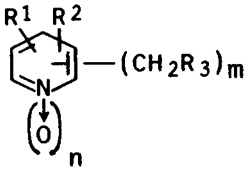
(II),

in der $R^1$, $R^2$, $R^3$ und m die obengenannten Bedeutungen haben und n für 0 oder 1 steht, mit einem Carbonsäureanhydrid der Formel $(R^4CO)_2O$, wobei $R^4$ die obengenannten Bedeutungen hat, in Gegenwart eines Übergangsmetallsalzes elektrochemisch umsetzt.

2. Verfahren zur Herstellung von Pyridinacylalen nach Anspruch 1, dadurch gekennzeichnet, daß man die elektrochemische Oxidation in Gegenwart eines zusätzlichen Hilfselektrolyten und/oder eines Colösungsmittels $R^4COOH$, wobei $R^4$ die in Anspruch 1 genannte Bedeutung hat, durchführt.

3. Verfahren zur Herstellung von Pyridinacylalen nach Anspruch 1, dadurch gekennzeichnet, daß man die Oxidation bei Temperaturen von 40 bis 150 °C durchführt.

4. Verfahren zur Herstellung von Pyridinacylalen nach Anspruch 1, dadurch gekennzeichnet, daß man die elektrochemische Oxidation an Graphitelektroden bei Stromdichten von 0,1 bis 200 $mA/cm^2$ durchführt.

**5.** Verwendung der Pyridinacylalen der allgemeinen Formel I zur Herstellung von Pyridincarbonylverbindungen oder deren N-Oxide der Formel III

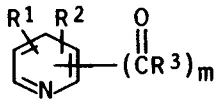

(III),

in der $R^1$, $R^2$ und $R^3$ und m die in Anspruch 1 genannten Bedeutungen haben.

Europäisches Patentamt

# EUROPÄISCHER RECHERCHENBERICHT

Nummer der Anmeldung

**EP 91 11 6653**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | CHEMICAL ABSTRACTS, vol. 114, no. 13, 1. April 1991, Columbus, Ohio, US; abstract no. 121994H, M. ABDEL AZZEM ET AL.: 'Indirect electrochemical oxidation of 4-picoline and its N-oxide to pyridine-4-aldehyde diacetate with electrogenated cobalt(III) acetate in an in-cell process' Seite 760 ; EP 91116653030 & Heterocycles 1990, 31(11), 1959-65 | 1 | C 07 D 213/51 |
| A,D | US-A-4 482 439 (J.E. TOOMEY, JR.) * Anspruch 1 * * | 1 | |
| A,D | JOURNAL OF THE AMERICAN CHEMICAL SOCIETY. Bd. 76, Nr. 5, 5. März 1954, GASTON, PA US Seiten 1286 - 1291; V. BOEKELHEIDE ET AL.: 'Rearrangement of N-Oxides. A Novel Synthesis of Pyridyl Carbinols and Aldehydes' * Seite 1287, letzter Absatz * * | 1 | |
| D,X | LIEBIGS ANNALEN DER CHEMIE. 1982, WEINHEIM DE Seiten 1615 - 1622; W. HASS ET AL.: 'Synthese der N-terminalen Aminosäure de Nikkomycine I, J, X und Z' * Seite 1616 * * | 5 | **RECHERCHIERTE SACHGEBIETE (Int. Cl.5)** |
| D,X | KHIMIYA GETEROTSIKLICHESKIKH SOEDINENII Bd. 1, Nr. 5, 1965, Seiten 499 - 502; N.S. PROSTAKOV ET AL.: 'Substituted pyridines. 5-Methyl-2-formylpyridine and 5-Methyl-4-phenyl-2-formylpyridine' * Seite 500, unten, Formelschema * * | 5 | C 07 D |
| A,D | HETEROCYCLES Bd. 5, 1976, Seiten 331 - 337; H. SAITO ET AL.: 'A novel oxidation of quinaldine N-oxide' * Seite 333, Formelschema * * | 5 | |

Der vorliegende Recherchenbericht wurde für alle Patentansprüche erstellt

| Recherchenort | Abschlußdatum der Recherche | Prüfer |
|---|---|---|
| Berlin | 17 Januar 92 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument